(19) Europäisches Patentamt
European Patent Office
Office européen des brevets

(11) Publication number: **0 413 785 B1**

# (12) EUROPEAN PATENT SPECIFICATION

(45) Date of publication of patent specification: **20.12.95**

(51) Int. Cl.[6]: **C09K 19/44**, C09K 19/46, C09K 19/10, C09K 19/30, C07C 25/18, C07C 43/225, C07C 43/174, C07C 69/75, C07C 69/773, C07C 69/035, G02F 1/13

(21) Application number: **90902191.7**

(22) Date of filing: **03.02.90**

(86) International application number: **PCT/EP90/00186**

(87) International publication number: **WO 90/09420 (23.08.90 90/20)**

## (54) LIOUID CRYSTALLINE MEDIA CONTAINING FLUORINATED OLIGOPHENYLS

(30) Priority: **17.02.89 GB 8903622**
**06.09.89 GB 8920161**

(43) Date of publication of application: **27.02.91 Bulletin 91/09**

(45) Publication of the grant of the patent: **20.12.95 Bulletin 95/51**

(84) Designated Contracting States: **CH DE FR GB IT LI NL**

(56) References cited:
**EP-A- 0 167 912**
**EP-A- 0 181 601**
**EP-A- 0 261 614**
**EP-A- 0 281 424**
**WO-A-87/03870**
**WO-A-88/02018**
**WO-A-88/08441**
**DE-A- 3 732 284**
**GB-A- 2 162 515**
**US-A- 4 780 240**
**US-A- 4 816 179**

(73) Proprietor: **MERCK PATENT GmbH**
**Postfach,**
**Frankfurter Strasse 250**
**D-64271 Darmstadt (DE)**

(72) Inventor: **COATES, David**
**87 Sopwith Crescent**
**Merley Wimborne**
**Dorset BH21 3SW (GB)**
Inventor: **GREENFIELD, Simon**
**2 Blackbird Close**
**Creekmoor**
**Poole BH17 7YA (GB)**
Inventor: **SMITH, Graham**
**10 Henbury Close**
**Canford Heath**
**Poole**
**Dorset BH17 8AX (GB)**
Inventor: **KURMEIER, Hans-Adolf**
**Hinter der Schule 3 a**
**D-6104 Seeheim-Jugenheim (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**Patent Abstracts of Japan, Vol 11, No 24, C399, abstract of JP 61-197670, publ 1986-09-01 CANON INC.**

Inventor: **DORSCH, Dieter**
**Reuterallee 24**
**D-6100 Darmstadt (DE)**

## Description

The invention relates to liquid cryastlline media being a mixture of at least 2 compounds, characterized in that at least one compound is a fluorinated oligophenyl according to formula I

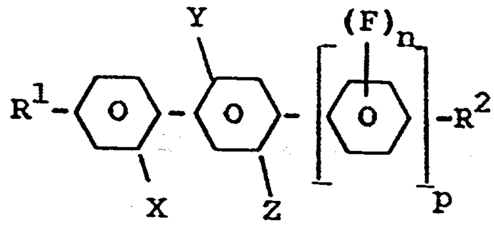

I

wherein
one of the groups $R^1$ and $R^2$ is F, $OCF_3$, $OCHF_2$ oder NCS and the other group is independently from the first group alkyl with 1 to 14 C atoms, wherein 1 or 2 $CH_2$ groups can be replaced by -O-, -O-CO-, -CO-O- and/or -CH=CH- with the proviso that two O atoms are not directly linked,
p is 0, 1 or 2 with the proviso that in case p = O at least one of X, Y and Z is F,
X, Y and Z each denote independently from each other H or F,
n is 0 or 1 with the proviso that in case n = 0 and p = 1 or 2 at least one of the groups X, Y and Z is F, and
Liquid crystalline media according to the invention containing one or more compounds of formula I wherein in case n = 1, p = 1 and $R^2$ = $OCF_3$

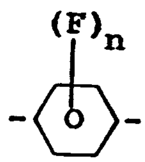

denotes

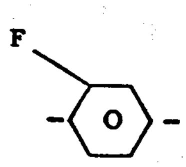

and in case $R^2$ = F, p = O and Z = F at least one of X and Y denotes F.

In formula I

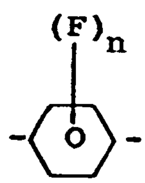

denotes in case n = 0 an unsubstituted 1,4 phenylene group and in case n = 1 a 1,4 phenylene group which is laterally monofluorinated in position 2 or 3.

The invention was based on the object of discovering new stable liquid crystal or mesogenic compounds which are suitable as components of liquid crystal phases and, in particular, have advantageous values for optical and dielectric anisotropy.

3,4-Difluorbiphenyls exhibiting a comparatively low viscosity are described in the Japanese laid-open documents JP 59-59636 and JP 61-207359. These biphenyls, however, have a rather small mesophase range. Trifluoromethoxy-2-fluoro-4-(4'-alkoxybiphenyl-4-yl)-benzenes are disclosed in DE 37 32 284. These compounds, however, exhibit rather poor values for $\Delta\epsilon/\epsilon_{\perp}$.

It has now been found that fluorinated oligophenyls of formulae I are highly suitable as components of liquid crystal media. In particular, they have especially advantageous values of the dielectric anisotropy $\Delta\epsilon$, relatively high or high values of the optical anisotropy $\Delta n$ and

they exhibit a high temperature and UV stability. It is also possible to obtain stable liquid crystal phases with a broad nematic mesophase range and a comparatively low viscosity with the aid of these compounds.

Depending on the choice of the substituents, the compounds of the formulae I can be used as the base materials from which liquid crystal media are predominantly composed; however, it is also possible for compounds of the formulae I to be added to liquid crystal base materials of other classes of compounds, for example in order to influence the dielectric and/or optical anisotropy and/or the viscosity and/or the mesophase range of such a dielectric.

The compounds of the formulae I are colourless in the pure state and are liquid crystalline in a temperature range which is favourably placed for electrooptical use. They are very stable towards chemicals, heat and light.

The invention thus relates to liquid crystalline media with at least two liquid crystalline components, wherein at least one component is a compound of the formula I and to liquid crystal display devices containing such media.

Furthermore, the invention relates to compounds of the formula I.

Above and below, $R^1$, $R^2$, X, Y, Z, and n have the meaning given unless expressly indicated otherwise.

The compounds of formula I include laterally mono- or polyfluorinated bi-, ter- and quaterphenyls, the bi- and terphenyls being preferred.

The following biphenyls are preferred:

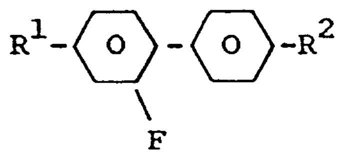

Ia1

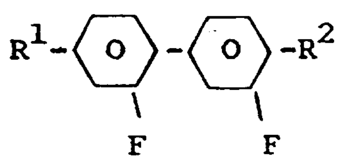

Ia2

Especially preferred are those compounds of formula Ia1 with $R^1$ being F, $OCF_3$ or $OCHF_2$ and $R^2$ denoting alkyl, alkenyl, alkoxy or alkanoyloxy and, moreover, those compounds with $R^2$ being F, $OCF_3$ or $OCHF_2$ and $R^1$ being n-alkyl, n-alkoxy or alkanoyloxy.

In formula Ia2 the polar group particularly is F or $OCF_3$.

The compounds of the formula I further include laterally mono- or polyfluorinated compounds with 3 rings. The compounds of formulae Ib1 - Ib4 are preferred:

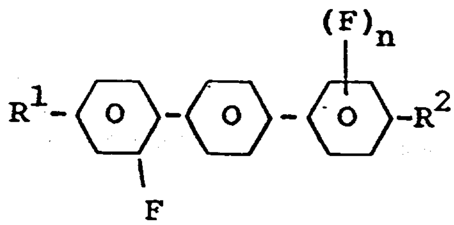

Ib1

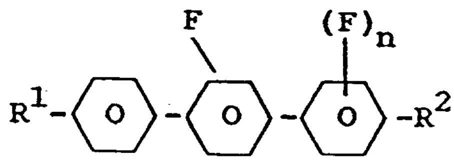

Ib2

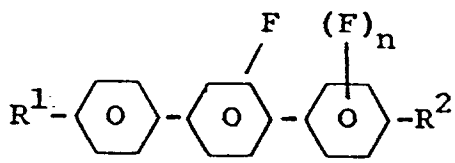

Ib3

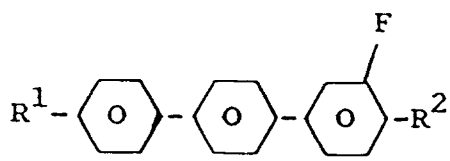

Ib4

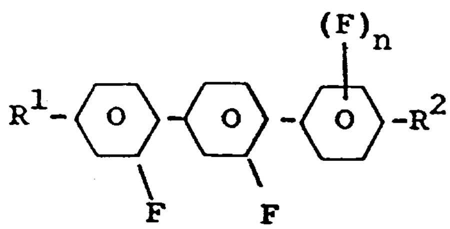

Ib5

The monofluorinated compounds of formula Ib21 are preferred:

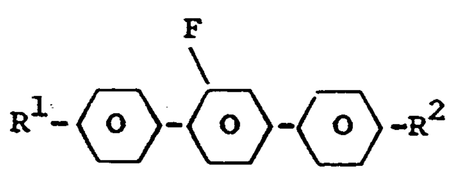

Ib21

Especially preferred are those compounds of formula Ib21 with $R^1$ denoting F, $OCF_3$, $OCHF_2$, or NCS and $R^2$ being n-alkyl or n-alkoxy with 1-9 C atoms or, in particular, n-alkyl with 2-7 C atoms.

The 3,4-difluorinated compounds of formula Ib41

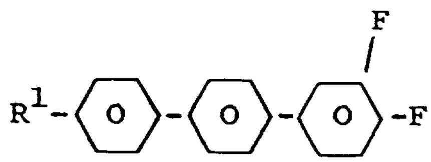

Ib41

are preferred; in this formula $R^1$ preferably is alkyl, alkoxy or alkanoyloxy and, in particular, n-alkyl or n-alkoxy with 1-9 C atoms. Moreover, the following laterally monofluorinated compunds of formula Ib11 are preferred:

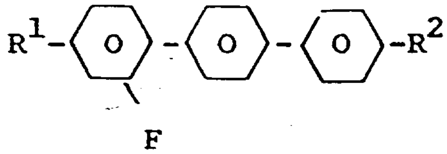

Ib11

Especially preferred are those compound of formula Ib11 with $R^1$ being F, $OCF_3$ or $OCHF_2$ and, in particular, F or $OCF_3$ and with $R^2$ denoting alkyl, alkoxy or alkanoyloxy with 1-9 C atoms and, moreover, those compounds with $R^2$ being F, $OCF_3$ or $OCHF_2$ and $R^1$ being n-alkyl, n-alkoxy or alkanoyloxy.

Moreover the following laterally bifluorinated compounds of formula Ib41 are preferred:

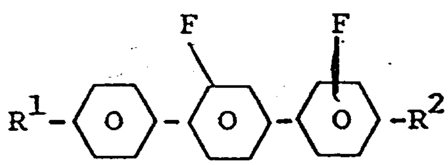

Ib22

Especially preferred are those copounds of formula Ib22 with $R^1$ = F or $OCF_3$ and

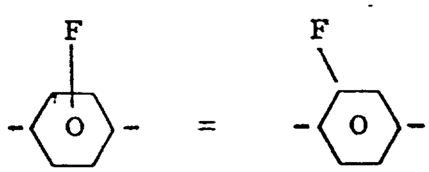

or

with $R^2$ = F, $OCHF_2$ or $OCF_3$ and $R^1$ = n-alkyl or n-alkoxy with 1-9 C atoms.

Further the following laterally bifluorinated compounds according to formula Ib31 are preferred:

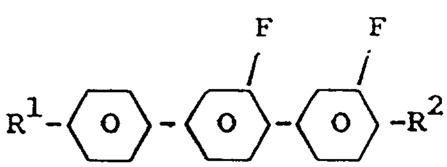

Ib31

The compounds of the formula I further include laterally mono-or polyfluorinated compounds with 4 rings. The compounds of the part formulae Ic1-Ic3 are preferred.

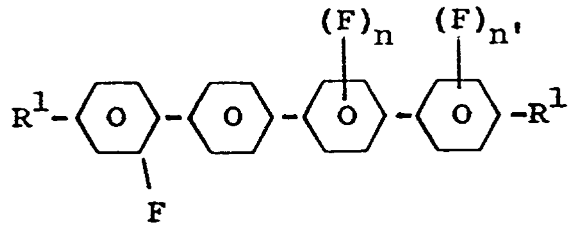

Ic1

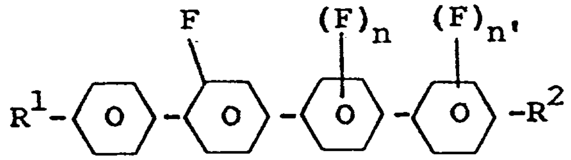

Ic2

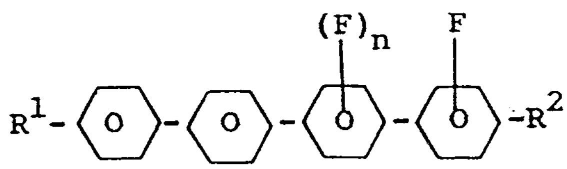

Ic3

In the part formulae Ic1-Ic3 n and n' denote independantly from each other 0 or 1.

The compounds of the formulae Ic1-Ic3 which are laterally mono- or difluorinated are preferred.

In laterally higher fluorinated compounds the terminal group which is not F, $OCF_3$, $OCH_{F2}$ or NCS preferably means alkyl or alkoxy.

Alkyl or alkoxy preferably are straight-chain and have 2, 3, 4, 5, 6 or 7 C atoms. Accordingly they are preferably ethyl, propyl, butyl, pentyl, hexyl, heptyl, ethoxy, propoxy, butoxy, pentoxy, hexoxy or heptoxy, also methyl, octyl, nonyl, decyl, undecyl, dodecyl, tridecyl, tetradecyl, pentadecyl, methoxy, octoxy, nonoxy, decoxy, undecoxy, dodecoxy, tridecoxy or tetradecoxy.

Oxaalkyl is preferably straight-chain 2-oxapropyl (= methoxymethyl), 2-(= ethoxymethyl) or 3-oxybutyl (= 2-methoxyethyl), 2-, 3- or 4-oxapentyl, 2-, 3-, 4- or 5-oxahexyl, 2-, 3-, 4- 5- or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-, 7-, 8-, or 9-oxadecyl.

Alkenyl is preferably straight-chain and has 2 or 10 C atoms. It is accordingly, in particular, vinyl, prop-1- or prop-2-enyl, but-1-, -2- or -3-enyl, pent-1-, -2-, -3- or -4-enyl, hex-1-, -2-, -3-, -4- or -5-enyl, hept-1-, -2-, -3-, -4-, -5- or -6-enyl, oct-1-, -2-, -3-, -4-, -5- -6- or -7-enyl, non-1-, -2-, -3-, -4-, -5-, -6-, -7- or -8-enyl or dec-1-, -2-, -3-, -4-, -5-, -6-, -7-, -8- or -9-enyl.

Compounds of the formula I containing a branched terminal group can occasionally be of importance because of an improved solubility in the customary liquid crystal base materials, but in particular as chiral doping substances if they are optically active. Smectic compounds of this type are suitable as components of ferro-electric materials.

Branched groups of this type as a rule contain not more than one chain branching. Preferred branched radicals are isopropyl, 2-butyl (= 1-methylpropyl), isobutyl (= 2-methylpropyl, 2-methylbutyl, isopentyl, (= 3-methylbutyl), 2-methylpentyl, 2-ethylhexyl, 2-propylpentyl, 2-octyl, isopropoxy, 2-methylpropoxy, 2-methylbutoxy, 3-methylbutoxy, 2-methylpentoxy, 3-methylpentoxy, 2-ethylhexoxy, 2-methylhexoxy, 1-methylhexoxy, 1-methylheptoxy (= 2-octyloxy), 2-oxa-3-methylbutyl, 3-oxa-4-methylpentyl, 4-methylhexyl, 2-nonyl, 2-decyl, 2-dodecyl, 6-methyloctoxy, 6-methyloctanoyloxy, 4-methylheptyloxycarbonyl, 2-methylbutyryloxy, 3-methylvaleryloxy, 4-methylhexanoyloxy, 2-chloro-4-methylvaleryloxy, 2-chlor-3-methylvaleryloxy, 2-methyl-3-oxapentyl and 2-methyl-3-oxahexyl.

In the case of compounds with a branched terminal group, formula I includes both the optical antipodes and racemates as well as mixtures thereof.

Of the compounds of the formula I and subformulae thereof, those in which at least one of the radicals contained therein has one of the preferred meanings given are preferred.

The compounds of the formula I are prepared by methods which are known per se, such as are described in the literature (for example in the standard works, such as Houben-Weyl, Methoden her Organischen Chemie [Methods of Organic Chemistry], Georg Thieme Verlag, Stuttgart), and in particular under reaction conditions which are known and suitable for the reactions mentioned. Variants which are known per se and are not mentioned in more detail here can also be used in this connection.

If desired, the starting materials can also be formed in situ, such that they are not isolated from the reaction mixture but are immediately reacted further to give the compounds of the formula I.

The compounds of formula I can be prepared, for example, by coupling mono- or polyphenyl boronic acids

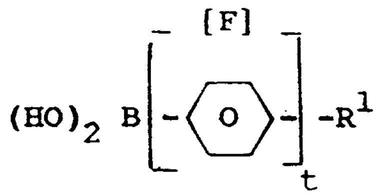

III

with 4-bromomono- or polyphenyls

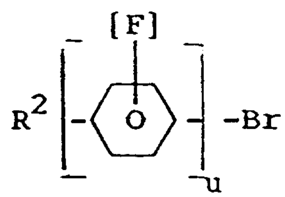

IV

with $t + u = p + 2$ and $R^1$ and $R^2$ having the meaning given above. Both reactants of formulae III and IV are laterally fluorinated appropriately to give compounds of formula I;
this is indicated schematically by the formula

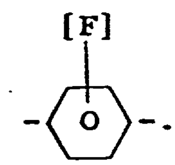

The compounds of formula I with $R_1$ or $R_2$ being $OCF_3$, NCS or F can further be prepared, for example, by coupling Zn mono- or polyphenyls

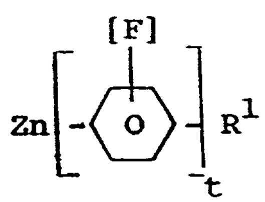

V

with 4-bromomono- or polyphenyls of formula IV via transition metal catalysis t, u, $R^1$, $R^2$ and

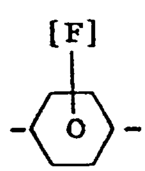

having the meaning indicated. This reaction is described in great detail in E. Poetsch, Kontakte, 1988(2), 15, Darmstadt.

In addition to one or more compounds of formula I the liquid crystal media according to the invention preferably contain 2-40 components and in particular 4-30 components. Liquid crystal media being composed of one or more compounds of formula I and 7-25 other components are especially preferred.

These additional components are preferably chosen from the nematic or nematogenic (monotropic or isotropic) substances; in particular from the classes of azoxybenzenes, benzylideneanilines, biphenyls, terphenyls, phenyl or cyclohexyl benzoates, phenyl or cyclohexyl cyclohexanecarboxylates, phenyl or cyclohexyl cyclohexylbenzoates, phenyl or cyclohexyl cyclohexylcyclohexanecarboxylates, cyclohexylphenylbenzoates, cyclohexylphenyl cyclohexanecarboxylates, cyclohexylphenyl cyclohexylcyclohexanecarboxylates, phenylcyclohexanes, cyclohexylbiphenyls, phenylcyclohexylcyclohexanes, cyclohexylcyclohexanes, cyclohexylcyclohexenes, cyclohexylcyclohexylcyclohexene, 1,4-bis-cyclohexylbenzenes, 4,4'-bis-cyclohexylbiphenyls, phenyl- or cyclohexylpyrimidines, phenyl- or cyclohexylpyridines, phenyl- or cyclohexyldioxanes, phenyl- or cyclohexyl-1,3-dithianes, 1,2-diphenylethanes, 1,2-dicyclohexylethanes, 1-phenyl-2-cyclohexylethanes,1-cyclohexyl-2-(4-phenyl-cyclohexyl)-ethanes, 1-cyclohexyl-2-biphenylethanes, 1-phenyl-2-cyclohexyl-phenylethanes, optionally halogenated stilbenes, benzyl phenyl ethers, tolanes and substituted cinnamic acids.

The 1,4-phenylene groups of these compounds may be fluorinated.

The most important compounds which are possible constituents of liquid crystal media according to the invention can be characterized by the formalae 1, 2, 3, 4 and 5:

R'-L-U-R''   1

R'-L-COO-U-R''   2

R'-L-OOC-U-R''   3

R'-L-$CH_2CH_2$-U-R''   4

R'-L-C≡C-U-R''   5

In the formulae 1, 2, 3, 4 and 5 L and U may be equal or different from each other. L and U independently from each other denote a bivalent residue selected from the group consisting of -Phe-, -Cyc-, -Phe-Phe-, -Phe-Cyc-, -Cyc-Cyc-, -Pyr-, -Dio-, -G-Phe-, -G-Cyc- and their mirror images; in this compilation of residues Phe denotes unsubstituted or fluorinated 1,4-phenylen, Cyc trans- 1,4-cyclohexylene or 1,4-cyclohexenylen, Pyr pyrimidine-2,5-diyl or pyridine-2,5-diyl, Dio 1,3-dioxane-2,5-diyl and G 2-(trans-1,4-cyclohexyl)-ethyl, pyrimidine-2,5-diyl, pyridine-2,5-diyl or 1,3-dioxane-2,5-diyl.

One of the residues L and U is preferably Cyc, Phe or Pyr. U Preferably denotes Cyc, Phe or Phe-Cyc. The liquid crystal media according to the invention preferably contain one or more components selected from the compounds of formulae 1, 2, 3, 4 and 5 with L and U meaning Cyc, Phe and Pyr, said liquid crystal media further containing at the same time one ore more components selected from the compounds of formulae 1, 2, 3, 4 and 5 with one of the residues L and U denoting Cyc, Phe and Pyr and the other residue being selected from the group consisting of -Phe-Phe-, -Phe-Cyc-, -Cyc-Cyc-, -G-Cyc-, said liquid crystal media containing in addition to this optionally one or more components selected from the compounds of formulae 1, 2, 3, 4 and 5 with L and U being selected from the group consisting of -Phe-Cyc-, -Cyc-Cyc-, -G-Phe- and -G-Cyc.

In a preferred subgroup of the compounds of formulae 1, 2, 3, 4 and 5 (subgroup 1) R' and R'' are independently from each other alkyl, alkenyl, alkoxy, alkenoxy with up to 8 carbon atoms. R' and R'' differ from one another in most of these compounds, one of the residues usually being alkyl or alkenyl. In another preferred subgroup of the compounds of formulae 1, 2, 3, 4 and 5 (subgroup 2) R'' denotes -CN, $-CF_3$, -F, -Cl or -NCS while R' has the meaning indicated in subgroup 1 and is preferably alkyl or alkenyl. Other variants of the envisaged substituents in the compounds of formulae 1, 2, 3, 4 and 5 are also customary. Many such substances are commercially available. All these substances are obtainable by methods which are known from the literature or by analogous methods.

The liquid crystal media according to the invention preferably contain in addition to components selected from subgroup 1 also components of subgroup 2, the percentage of these components being as follows:

subgroup 1:   20 to 90 %, in particular 30 to 90 %

subgroup 2: 10 to 50 %, in particular 10 to 50 %

In these liquid crystal media the percentages of the compounds according to the invention and the compounds of subgroup 1 and 2 may add up to give 100 %.

The media according to the invention preferably contain 1 to 40 %, in particular 5 to 30 % of the compounds according to the invention. Media containing more than 40 %, in particular 45 to 90 % of the compounds according to the invention are further preferred. The media contain preferably 3, 4 or 5 compounds according to the invention.

Liquid crystalline media according to the invention containing one or more components selected from the group comprising compounds of the furmlae Ia1, Ia2, Ib1, Ib2, Ib3, Ib4, Ib5, Ib21, Ib41, Ib11, Ib22, Ib31, Ic1, Ic2 and Ic3 are preferred.

Liquid crystalline media containing one or more compounds selected from the group comprising laterally fluorinated bi-, ter- and quaterphenyls of the formulae Ia1, Ia2, Ib1, Ib3, Ib4, Ib5, Ib21, Ib41, Ib11, Ib31, Ib22, Ic1, Ic2 and Ic3 are preferred. Especially preferred are liquid crystalline media one or more components of which are selected from a smaller subgroup consisting of compounds of formulae Ia1, Ia2, Ib1, Ib2, Ib3, Ib4, Ib21, Ib41, Ib11, Ib22, Ib31 and Ic3.

Liquid crystalline media according to the invention are characterized by advantageous values of the dielectric anisotropy $\Delta\epsilon$ and the viscosity $\eta$, by a high clearing point $T_c$ and a wide nematic range and, in particular, by advantageous values of the optical anisotropy $\Delta n$ and a high UV and temperature stability. The liquid crystalline media according to the invention can preferably be used, for example, in twisted nematic (TN) cells, especially in STN (supertwisted nematic) cells with a twist angle > 90 °, further in electrooptical systems consisting of nematic microdroplets embedded in a transparent matrix like for example PDLC (polymer dispersed liquid crystal) or NCAP (nematic curvilinear aligned phase) devices or in electrooptical PN (polymer network) systems which are described, for example, in EP 0,313,053. Further, the media can preferably be used for active-matrix-adressed TN cells like TET-TN (thin-film-transistar TN) or MIM-TN (metal-insulator-metal TN) cells, especially, for example, for projection systems or other displays being operated under the so-called 2. minimum-condition or with comparable or even higher values of d . $\Delta n$.

The media according to the invention are prepared in a manner which is customary per se. As a rule, the components are dissolved in one another, advantageously at elevated temperature. The liquid crystal media according to the invention can be modified by suitable additives so that they can be used in all the types of liquid crystal display devices. Such additives are known to the expert and are described in detail in the literature (H. Kelker/R. Hatz, Handbook of Liquid Crystals, Verlag Chemie, Weinheim, 1980). For example, it is possible to add pleochroic dyestuffs to prepare colored guest-host systems or substances for modifying the dielectric anisotropy, the viscosity and/or the orientation of the nematic phases.

The following examples are to be construed as merely illustrative and not limitative. m.p. = melting point, c.p. = clearing point. In the foregoing and in the following all parts and percentages are by weight and the temperatures are set forth in degrees Celsius. "Customary work-up" means that water is added, the mixture is extracted with methylene chloride, the organic phase is seperated off, dried and evaporated, and the product is purified by crystallization and/or chromatography.

Further are:

C: crystalline-solid state, S: smectic phase (the index denoting the typ of smectic phase), N: nematic phase, Ch: cholesteric phase, I: isotropic phase. The number being embraced by 2 of these symbols denotes the temperature of phase change.

Examples for production

Example 1

Step 1: 0.22 mol 4'-pentyl-4-bromobiphenyl is dissolved in 200 ml tetrahydrofuran and metallized by reaction with 0.22 mol magnesium turnings in a nitrogen atmosphere. 0.22 mol trimethyl borate is added dropwise. Addition of 150 ml of 20 % hydrochloric acid solution and customary work-up give 4-pentylbiphenyl-4'-boronic acid.

Step 2: 0.44 mol 4-pentylbiphenyl-4'-boronic acid, 0.036 mol 1-bromo-2,4-difluorobenzene, 70 ml toluene, 20 ml industrial methylated spirits and 35 ml 2m sodium carbonate are mixed under nitrogen and stirred and heated under reflux with 0.5 g tetra-(triphenylphosphine)-palladium for 16 hours. Customary work-up gives 2,4-difluoro-4''-pentylterphenyl exhibiting the follwoing data: C 82.2 $S_A$ 122.9 N 133.2 I.

The following compounds are prepared analogously:
2,4-difluoro-4''-ethylterphenyl
2,4-difluoro-4''-propylterphenyl
2,4-difluoro-4''-butylterphenyl
2,4-difluoro-4''-heptylterphenyl
2,4-difluoro-4''-decylterphenyl
2,4-difluoro-4''-ethoxyterphenyl
2,4-difluoro-4''-butoxyterphenyl
2,4-difluoro-4''-propoxyterphenyl
2,4-difluoro-4''-pentoxyterphenyl
2,4-difluoro-4''-hexoxyterphenyl
2,4-difluoro-4''-heptoxterphenyl
2,4-difluoro-4''-(2-oxapentyl)-terphenyl
2,4-difluoro-4''-(3-oxapentyl)-terphenyl
2,4-difluoro-4''-(4-oxapentyl)-terphenyl
2,4-difluoro-4''-(2-methylpentyl)-terphenyl
2,4-difluoro-4''-(2-decyl)-terphenyl
2,4-difluoro-4''-(2-ethylhexoxy)-terphenyl
3,4-difluoro-4''-ethylterphenyl
3,4-difluoro-4''-propylterphenyl
3,4-difluoro-4''-butylterphenyl
3,4-difluoro-4''-pentylterphenyl C 154 $S_A$ 163 I
3,4-difluoro-4''-hexylterphenyl
3,4-difluoro-4''-heptylterphenyl
3,4-difluoro-4''-nonylterphenyl
3,4-difluoro-4''-ethoxyterphenyl
3,4-difluoro-4''-propoxyterphenyl
3,4-difluoro-4''-butoxyterphenyl
3,4-difluoro-4''-pentoxyterphenyl
3,4-difluoro-4''-heptoxyterphenyl
3,4-difluoro-4''-(hex-3-enyl)-terphenyl
3,4-difluoro-4''-(hex-5-enyl)-terphenyl
3,4-difluoro-4''-(hept-3-enyl)-terphenyl
3,4-difluoro-4''-(hept-6-enyl)-terphenyl
3,4-difluoro-4''-(3-oxaheptyl)-terphenyl
3,4-difluoro-4''-(5-oxaheptyl)-terphenyl
3,4-difluoro-4''-(6-oxaheptyl)-terphenyl
3,4-difluoro-4''-(7-oxaheptyl)-terphenyl

Example 2

4-fluoro-2'-fluoro-4''-propylterphenyl is prepared analogously to example 1 from 4-fluorophenyl boronic acid and 4-bromo-2-fluoro-4'-propylbiphenyl. This compound exhibits the following physical data: C 98.3 N 122.8 I

The following compounds are prepared analogously:
4-fluoro-2'-fluoro-4''-ethylterphenyl C 108 N 113.6 I
4-fluoro-2'-fluoro-4''-pentylterphenyl
4-fluoro-2'-fluoro-4''-heptylterphenyl
4-fluoro-2'-fluoro-4''-nonylterphenyl
4-fluoro-2'-fluoro-4''-pentoxyterphenyl
4-fluoro-2'-fluoro-4''-heptoxyterphenyl
4-fluoro-2'-fluoro-4''-nonoxyterphenyl
4-fluoro-2',5'-difluoro-4''-ethylterphenyl
4-fluoro-2',5'-difluoro-4''-propylterphenyl
4-fluoro-2',5'-difluoro-4''-butylterphenyl
4-fluoro-2',5'-difluoro-4''-pentylterphenyl
4-fluoro-2',5'-difluoro-4''-hexylterphenyl
4-fluoro-2',5'-difluoro-4''-heptylterphenyl

4-fluoro-2',5'-difluoro-4''-octylterphenyl
4-fluoro-2',5'-difluoro-4''-nonylterphenyl
4-fluoro-2',5'-difluoro-4''-decylterphenyl
4-fluoro-2',5'-difluoro-4''-butoxyterphenyl
4-fluoro-2',5'-difluoro-4''-pentoxyterphenyl
4-fluoro-2',5'-difluoro-4''-hexoxyterphenyl
4-fluoro-2',5'-difluoro-4''-heptoxyterphenyl
4-fluoro-2',5'-difluoro-4''-octoxyterphenyl
4-fluoro-2',5'-difluoro-4''-nonoxyterphenyl
4-fluoro-2',5'-difluoro-4''-decoxyterphenyl
4-fluoro-2',5'-difluoro-4''-(hex-3-enyl)-terphenyl
4-fluoro-2',5'-difluoro-4''-(hex-5-enyl)-terphenyl
4-fluoro-2',5'-difluoro-4''-(hept-3-enyl)-terphenyl
4-fluoro-2',5'-difluoro-4''-(hept-5-enyl)-terphenyl

Example 3

4-fluoro-2''-fluoro-4''-propylterphenyl is prepared analogously to example 1 from 4-fluorophenyl boronic acid and 4-bromo-2'-fluoro-4'-propylbiphenyl. The following compounds are prepared analogously:
4-fluoro-2''-fluoro-4''-methylterphenyl
4-fluoro-2''-fluoro-4''-ethylterphenyl
4-fluoro-2''-fluoro-4''-butylterphenyl
4-fluoro-2''-fluoro-4''-pentylterphenyl
4-fluoro-2''-fluoro-4''-hexylterphenyl
4-fluoro-2''-fluoro-4''-heptylterphenyl
4-fluoro-2''-fluoro-4''-octylterphenyl
4-fluoro-2''-fluoro-4''-nonylterphenyl
4-fluoro-2''-fluoro-4''-decylterphenyl
4-fluoro-2''-fluoro-4''-methoxyterphenyl
4-fluoro-2''-fluoro-4''-propoxyterphenyl
4-fluoro-2''-fluoro-4''-butoxyterphenyl
4-fluoro-2''-fluoro-4''-pentoxyterphenyl
4-fluoro-2''-fluoro-4''-hexoxyterphenyl
4-fluoro-2''-fluoro-4''-oxtoxyterphenyl

Example 4

3,4-difluoro-2'-fluoro-4''-propylterphenyl is prepared analogously to example 1 from 3,4-difluorophenyl boronic acid and 4-bromo-2-fluoro-4'-propylphenyl
The following compounds are prepared analogously:
3,4-difluoro-2'-fluoro-4''-ethylterphenyl
3,4-difluoro-2'-fluoro-4''-butylterphenyl
3,4-difluoro-2'-fluoro-4''-pentylterphenyl
3,4-difluoro-2'-fluoro-4''-hexylterphenyl
3,4-difluoro-2'-fluoro-4''-heptylterphenyl
3,4-difluoro-2'-fluoro-4''-octylterphenyl
3,4-difluoro-2'-fluoro-4''-nonylterphenyl
3,4-difluoro-2'-fluoro-4''-decylterphenyl
3,4-difluoro-2'-fluoro-4''-butoxyterphenyl
3,4-difluoro-2'-fluoro-4''-pentoxyterphenyl
3,4-difluoro-2'-fluoro-4''-hexoxyterphenyl
3,4-difluoro-2'-fluoro-4''-heptoxyterphenyl
3,4-difluoro-2'-fluoro-4''-octoxyterphenyl
3,4-difluoro-2'-fluoro-4''-nonoxyterphenyl
3,4-difluoro-2'-fluoro-4''-decoxyterphenyl
3,4-difluoro-2'-fluoro-4''-(but-3-enyl)-terphenyl
3,4-difluoro-2'-fluoro-4''-(pent-4-enyl)-terphenyl
3,4-difluoro-2'-fluoro-4''-(hex-5-enyl)-terphenyl

Example 5

2,4-difluoro-4'-propylbiphenyl is prepared analogously to example 1 from 2,4-difluorophenyl boronic acid and 4-bromo-1-propylbenzene.

The following compounds are prepared analogously:

2,4-difluoro-4'-pentylbiphenyl
2,4-difluoro-4'-hexylbiphenyl
2,4-difluoro-4'-heptylbiphenyl
2,4-difluoro-4'-octylbiphenyl
2,4-difluoro-4'-nonylbiphenyl
2,4-difluoro-4'-decylbiphenyl
2,4-difluoro-4'-ethoxybiphenyl
2,4-difluoro-4'-propoxybiphenyl C 57.6 I
2,4-difluoro-4'-butoxybiphenyl
2,4-difluoro-4'-pentoxybiphenyl
2,4-difluoro-4'-hexoxybiphenyl
2,4-difluoro-4'-heptoxybiphenyl
2,4-difluoro-4'-octoxybiphenyl
2,4-difluoro-4'-nonoxybiphenyl
2,4-difluoro-4'-decoxybiphenyl
2,4-difluoro-4'-(hex-3-enyl)-biphenyl
2,4-difluoro-4'-(hex-5-enyl)-biphenyl
2,4-difluoro-4'-(hept-3-enyl)-biphenyl
2,4-difluoro-4'-(hept-6-enyl)-biphenyl
2,4-difluoro-4'-isobutyl-biphenyl
2,4-difluoro-4'-(3-methylbutoxy)-biphenyl

Example 6

According to example 6 4'-fluoro-3-fluoro-4-acetoxybiphenyl is prepared from acetic acid and 4'-fluoro-3-fluoro-4-hydroxybiphenyl. This compound exhibits the following physical data: C 84.5 I

The following compounds are prepared analogously:

4'-fluoro-3-fluoro-4-propionyloxybiphenyl
4'-fluoro-3-fluoro-4-butyryloxybiphenyl;
clearing point < 20 °C
4'-fluoro-3-fluoro-4-valeryloxybiphenyl
4'-fluoro-3-fluoro-4-(6-methyloctanoyloxy)-biphenyl
4'-fluoro-3-fluoro-4-(2-methylbutyryloxy)-biphenyl
2',4'-difluoro-4-acetoxybiphenyl
2',4'-difluoro-4-propionyloxybiphenyl
2',4'-difluoro-4-valeryloxybiphenyl
2',4'-difluoro-4-hexanoyloxybiphenyl
2',4'-difluoro-4-(4-methylhexanoyloxy)-biphenyl

Example 7

Difluoromethoxy-3-fluoro-4-[4-propyl-biphenyl-4'-yl]-benzene is prepared analogously to example 1 from 4-difluoromethoxy-2-fluorophenyl boronic acid and 4-bromo-4'-propylbiphenyl.

The following compounds are prepared analogously:

Difluoromethoxy-3-fluoro-4-[4-methyl-biphenyl-4'-yl]-benzene
Difluoromethoxy-3-fluoro-4-[4-ethyl-biphenyl-4'-yl]-benzene
Difluoromethoxy-3-fluoro-4-[4-butyl-biphenyl-4'-yl]-benzene
Difluoromethoxy-3-fluoro-4-[4-pentyl-biphenyl-4'-yl]-benzene
Difluoromethoxy-3-fluoro-4-[4-hexyl-biphenyl-4'-yl]-benzene
Difluoromethoxy-3-fluoro-4-[4-heptyl-biphenyl-4'-yl]-benzene
Difluoromethoxy-3-fluoro-4-[4-methoxy-biphenyl-4'-yl]-benzene
Difluoromethoxy-3-fluoro-4-[4-ethoxy-biphenyl-4'-yl]-benzene
Difluoromethoxy-3-fluoro-4-[4-propoxy-biphenyl-4'-yl]-benzene

Difluoromethoxy-3-fluoro-4-[4-butoxy-biphenyl-4'-yl]-benzene
Difluoromethoxy-3-fluoro-4-[4-pentoxy-biphenyl-4'-yl]-benzene
Difluoromethoxy-3-fluoro-4-[4-hexoxy-biphenyl-4'-yl]-benzene
Difluoromethoxy-3-fluoro-4-[4-heptoxy-biphenyl-4'-yl]-benzene
Difluoromethoxy-2-fluoro-4-[4-ethyl-biphenyl-4'-yl]-benzene
Difluoromethoxy-2-fluoro-4-[4-propyl-biphenyl-4'-yl]-benzene
Difluoromethoxy-2-fluoro-4-[4-butyl-biphenyl-4'-yl]-benzene
Difluoromethoxy-2-fluoro-4-[4-pentyl-biphenyl-4'-yl]-benzene
Difluoromethoxy-2-fluoro-4-[4-hexyl-biphenyl-4'-yl]-benzene
Difluoromethoxy-2-fluoro-4-[4-heptyl-biphenyl-4'-yl]-benzene
Difluoromethoxy-2-fluoro-4-[4-ethoxy-biphenyl-4'-yl]-benzene
Difluoromethoxy-2-fluoro-4-[4-propoxy-biphenyl-4'-yl]-benzene
Difluoromethoxy-2-fluoro-4-[4-butoxy-biphenyl-4'-yl]-benzene
Difluoromethoxy-2-fluoro-4-[4-pentoxy-biphenyl-4'-yl]-benzene
Difluoromethoxy-2-fluoro-4-[4-hexoxy-biphenyl-4'-yl]-benzene
Difluoromethoxy-2-fluoro-4-[4-(hex-3-enyl)-biphenyl-4'-yl]-benzene
Difluoromethoxy-2-fluoro-4-[4-(hex-5-enyl)-biphenyl-4'-yl]-benzene
Difluoromethoxy-2-fluoro-4-[4-(hept-3-enyl)-biphenyl-4'-yl]-benzene
Difluoromethoxy-2-fluoro-4-[4-(hept-5-enyl)-biphenyl-4'-yl]-benzene
Difluoromethoxy-2-fluoro-4-[4-(hept-6-enyl)-biphenyl-4'-yl]-benzene

Example 8

Difluoromethoxy-4-[4-propyl-3'-fluoro-biphenyl)-4'-yl]-benzene is prepared analogously to example 1 from 4-difluoromethoxyphenyl boronic acid and 3-fluoro-4-bromo-4'-propylbiphenyl. This compound exhibits the following physical data: C 96 $S_A$ 153 I
The following compounds are prepared analogously:
Difluoromethoxy-4-[4-methyl-3'-fluoro-biphenyl-4'-yl]-benzene
Difluoromethoxy-4-[4-ethyl-3'-fluoro-biphenyl-4'-yl]-benzene
Difluoromethoxy-4-[4-butyl-3'-fluoro-biphenyl-4'-yl]-benzene
Difluoromethoxy-4-[4-pentyl-3'-fluoro-biphenyl-4'-yl]-benzene
Difluoromethoxy-4-[4-hexyl-3'-fluoro-biphenyl-4'-yl]-benzene
Difluoromethoxy-4-[4-heptyl-3'-fluoro-biphenyl-4'-yl]-benzene
Difluoromethoxy-4-[4-methoxy-3'-fluoro-biphenyl-4'-yl]-benzene
Difluoromethoxy-4-[4-ethoxy-3'-fluoro-biphenyl-4'-yl]-benzene
Difluoromethoxy-4-[4-propoxy-3'-fluoro-biphenyl-4'-yl]-benzene
Difluoromethoxy-4-[4-butoxy-3'-fluoro-biphenyl-4'-yl]-benzene
Difluoromethoxy-4-[4-pentoxy-3'-fluoro-biphenyl-4'-yl]-benzene
Difluoromethoxy-4-[4-heptoxy-3'-fluoro-biphenyl-4'-yl]-benzene
Difluoromethoxy-4-[4-(hex-3-enyl)-3'-fluoro-biphenyl-4'-yl]-benzene
Difluoromethoxy-4-[4-(hex-4-enyl)-3'-fluoro-biphenyl-4'-yl]-benzene
benzene
Difluoromethoxy-4-[4-(hex-5-enyl)-3'-fluoro-biphenyl-4'-yl]-benzene
Difluoromethoxy-4-[4-(2-methylpropyl)-3'-fluoro-biphenyl-4'-yl]-benzene
Difluoromethoxy-4-[4-(2-chloro-pentyl)-3'-fluoro-biphenyl-4'-yl]-benzene
Difluoromethoxy-4-[4-(3-chloro-pentyl)-3'-fluoro-biphenyl-4'-yl]-benzene
Difluoromethoxy-4-[4-methyl-2'-fluoro-biphenyl-4'-yl]-benzene
Difluoromethoxy-4-[4-ethyl-2'-fluoro-biphenyl-4'-yl]-benzene, $S_A$ 147 N 161 I
Difluoromethoxy-4-[4-propyl-2'-fluoro-biphenyl-4'-yl]-benzene
Difluoromethoxy-4-[4-butyl-2'-fluoro-biphenyl-4'-yl]-benzene
Difluoromethoxy-4-[4-pentyl-2'-fluoro-biphenyl-4'-yl]-benzene
Difluoromethoxy-4-[4-hexyl-2'-fluoro-biphenyl-4'-yl]-benzene
Difluoromethoxy-4-[4-heptyl-2'-fluoro-biphenyl-4'-yl]-benzene
Difluoromethoxy-4-[4-methoxy-2'-fluoro-biphenyl-4'-yl]-benzene
Difluoromethoxy-4-[4-ethoxy-2'-fluoro-biphenyl-4'-yl]-benzene
Difluoromethoxy-4-[4-propoxy-2'-fluoro-biphenyl-4'-yl]-benzene
Difluoromethoxy-4-[4-butoxy-2'-fluoro-biphenyl-4'-yl]-benzene
Difluoromethoxy-4-[4-pentoxy-2'-fluoro-biphenyl-4'-yl]-benzene

Difluoromethoxy-4-[4-hexoxy-2'-fluoro-biphenyl-4'-yl]-benzene
Difluoromethoxy-4-[4-heptoxy-2'-fluoro-biphenyl-4'-yl]-benzene

Example 9

Difluoromethoxy-4-[2-fluoro-4-ethylbiphenyl-4'-yl]-benzene is prepared analogously to example 1 from 4-difluoromethoxyphenyl boronic acid and 2-fluoro-4-ethyl-4'-bromobiphenyl.

The following compounds are prepared analogously:
Difluoromethoxy-4-[2-fluoro-4-methylbiphenyl-4'-yl]-benzene
Difluoromethoxy-4-[2-fluoro-4-propylbiphenyl-4'-yl]-benzene
Difluoromethoxy-4-[2-fluoro-4-butylbiphenyl-4'-yl]-benzene
Difluoromethoxy-4-[2-fluoro-4-pentylbiphenyl-4'-yl]-benzene
Difluoromethoxy-4-[2-fluoro-4-hexylbiphenyl-4'-yl]-benzene
Difluoromethoxy-4-[2-fluoro-4-heptylbiphenyl-4'-yl]-benzene
Difluoromethoxy-4-[2-fluoro-4-octylbiphenyl-4'-yl]-benzene
Difluoromethoxy-4-[2-fluoro-4-nonylbiphenyl-4'-yl]-benzene
Difluoromethoxy-4-[2-fluoro-4-decylbiphenyl-4'-yl]-benzene
Difluoromethoxy-4-[2-fluoro-4-methoxybiphenyl-4'-yl]-benzene
Difluoromethoxy-4-[2-fluoro-4-ethoxybiphenyl-4'-yl]-benzene
Difluoromethoxy-4-[2-fluoro-4-propoxybiphenyl-4'-yl]-benzene
Difluoromethoxy-4-[2-fluoro-4-butoxybiphenyl-4'-yl]-benzene
Difluoromethoxy-4-[2-fluoro-4-pentoxybiphenyl-4'-yl]-benzene
Difluoromethoxy-4-[2-fluoro-4-hexoxybiphenyl-4'-yl]-benzene
Difluoromethoxy-4-[2-fluoro-4-heptoxybiphenyl-4'-yl]-benzene
Difluoromethoxy-4-[2-fluoro-4-octoxybiphenyl-4'-yl]-benzene
Difluoromethoxy-4-[2-fluoro-4-(hex-3-enyl)biphenyl-4'-yl]-benzene
Difluoromethoxy-4-[2-fluoro-4-(hex-4-enyl)biphenyl-4'-yl]-benzene
Difluoromethoxy-4-[2-fluoro-4-(hex-5-enyl)biphenyl-4'-yl]-benzene

Example 10

Difluoromethoxy-2-fluoro-4-[4-propyl-3'-fluoro-biphenyl-4'-yl]-benzene is prepared analogously to example 1 from 4-difluoromethoxy-3-fluorophenyl boronic acid and 4-propyl-3'-fluoro-4'-bromobiphenyl.

The following compounds are prepared analogously:
Difluoromethoxy-2-fluoro-4-[4-methyl-3'-fluoro-biphenyl-4'-yl]-benzene
Difluoromethoxy-2-fluoro-4-[4-ethyl-3'-fluoro-biphenyl-4'-yl]-benzene
Difluoromethoxy-2-fluoro-4-[4-butyl-3'-fluoro-biphenyl-4'-yl]-benzene
Difluoromethoxy-2-fluoro-4-[4-pentyl-3'-fluoro-biphenyl-4'-yl]-benzene
Difluoromethoxy-2-fluoro-4-[4-hexyl-3'-fluoro-biphenyl-4'-yl]-benzene
Difluoromethoxy-2-fluoro-4-[4-heptyl-3'-fluoro-biphenyl-4'-yl]-benzene
Difluoromethoxy-2-fluoro-4-[4-methoxy-3'-fluoro-biphenyl-4'-yl]-benzene
Difluoromethoxy-2-fluoro-4-[4-ethoxy-3'-fluoro-biphenyl-4'-yl]-benzene
Difluoromethoxy-2-fluoro-4-[4-propoxy-3'-fluoro-biphenyl-4'-yl]-benzene
Difluoromethoxy-2-fluoro-4-[4-butoxy-3'-fluoro-biphenyl-4'-yl]-benzene
Difluoromethoxy-2-fluoro-4-[4-pentoxy-3'-fluoro-biphenyl-4'-yl]-benzene
Difluoromethoxy-2-fluoro-4-[4-hexoxy-3'-fluoro-biphenyl-4'-yl]-benzene
Difluoromethoxy-2-fluoro-4-[4-heptoxy-3'-fluoro-biphenyl-4'-yl]-benzene
Difluoromethoxy-2-fluoro-4-[4-oxtoxy-3'-fluoro-biphenyl-4'-yl]-benzene
Difluoromethoxy-2-fluoro-4-[4-monoxy-3'-fluoro-biphenyl-4'-yl]-benzene
Difluoromethoxy-2-fluoro-4-[4-decoxy-3'-fluoro-biphenyl-4'-yl]-benzene
Difluoromethoxy-2-fluoro-4-[4-methyl-2'-fluoro-biphenyl-4'-yl)-benzene
Difluoromethoxy-2-fluoro-4-[4-ethyl-2'-fluoro-biphenyl-4'-yl)-benzene
Difluoromethoxy-2-fluoro-4-[4-propyl-2'-fluoro-biphenyl-4'-yl)-benzene
Difluoromethoxy-2-fluoro-4-[4-butyl-2'-fluoro-biphenyl-4'-yl)-benzene
Difluoromethoxy-2-fluoro-4-[4-pentyl-2'-fluoro-biphenyl-4'-yl)-benzene
Difluoromethoxy-2-fluoro-4-[4-hexyl-2'-fluoro-biphenyl-4'-yl)-benzene
Difluoromethoxy-2-fluoro-4-[4-heptyl-2'-fluoro-biphenyl-4'-yl)-benzene
Difluoromethoxy-2-fluoro-4-[4-methoxy-2'-fluoro-biphenyl-4'-yl)-benzene

Difluoromethoxy-2-fluoro-4-[4-ethoxy-2'-fluoro-biphenyl-4'-yl)-benzene
Difluoromethoxy-2-fluoro-4-[4-propoxy-2'-fluoro-biphenyl-4'-yl)-benzene
Difluoromethoxy-2-fluoro-4-[4-butoxy-2'-fluoro-biphenyl-4'-yl)-benzene
Difluoromethoxy-2-fluoro-4-[4-pentoxy-2'-fluoro-biphenyl-4'-yl)-benzene
Difluoromethoxy-2-fluoro-4-[4-hexoxy-2'-fluoro-biphenyl-4'-yl)-benzene
Difluoromethoxy-2-fluoro-4-[4-heptoxy-2'-fluoro-biphenyl-4'-yl)-benzene
Difluoromethoxy-2-fluoro-4-[4-octoxy-2'-fluoro-biphenyl-4'-yl)-benzene
Difluoromethoxy-2-fluoro-4-[4-nonoxy-2'-fluoro-biphenyl-4'-yl)-benzene
Difluoromethoxy-2-fluoro-4-[4-decoxy-2'-fluoro-biphenyl-4'-yl)-benzene

Example 11

16.5 g of 4-pentyl-3'-fluoro-4'-bromobiphenyl in 50 ml of THF are added to a boiling mixture of 1.2 g of magnesium and 25 ml of THF. When the addition is complete, the mixture is heated for a further 1 hours, cooled and added to a solution of 6.7 g of $ZnBr_2$ in 50 ml of THF at 0°-15°. After stirring for 1 hour, 12.3 g of 1-bromo-4-(trifluoromethoxy)benzene and 0.75 g of $PdCl_2$ (dppf) are added. The mixture is stirred at 5 ° for 15 minutes and then at room temperature for 24 hours. The mixture is poured onto 100 ml of saturated $NH_4Cl$ solution, and the organic phase is separated off and extracted with toluene. After work-up of the organic phase and purification by chromatography and/or crystallization, trifluoromethoxy-4-[4-penty-3'-fluoro-biphenyl-4'-yl]-benzene is obtained.

The following compounds are prepared analogously:
Trifluoromethoxy-4-[-4-methyl-3'-fluoro-biphenyl-4'-yl]-benzene
Trifluoromethoxy-4-[-4-ethyl-3'-fluoro-biphenyl-4'-yl]-benzene
Trifluoromethoxy-4-[-4-propyl-3'-fluoro-biphenyl-4'-yl]-benzene
Trifluoromethoxy-4-[-4-butyl-3'-fluoro-biphenyl-4'-yl]-benzene
Trifluoromethoxy-4-[-4-hexyl-3'-fluoro-biphenyl-4'-yl]-benzene
Trifluoromethoxy-4-[-4-heptyl-3'-fluoro-biphenyl-4'-yl]-benzene
Trifluoromethoxy-4-[-4-octyl-3'-fluoro-biphenyl-4'-yl]-benzene
Trifluoromethoxy-4-[-4-nonyl-3'-fluoro-biphenyl-4'-yl]-benzene
Trifluoromethoxy-4-[-4-methoxy-3'-fluoro-biphenyl-4'-yl]-benzene
Trifluoromethoxy-4-[-4-propoxy-3'-fluoro-biphenyl-4'-yl]-benzene
Trifluoromethoxy-4-[-4-butoxy-3'-fluoro-biphenyl-4'-yl]-benzene
Trifluoromethoxy-4-[-4-pentoxy-3'-fluoro-biphenyl-4'-yl]-benzene
Trifluoromethoxy-4-[-4-hexoxy-3'-fluoro-biphenyl-4'-yl]-benzene
Trifluoromethoxy-4-[-4-heptoxy-3'-fluoro-biphenyl-4'-yl]-benzene
Trifluoromethoxy-4-[-4-octoxy-3'-fluoro-biphenyl-4'-yl]-benzene
Trifluoromethoxy-4-[-4-nonoxy-3'-fluoro-biphenyl-4'-yl]-benzene
Trifluoromethoxy-4-[-4-(hex-3-enyl)-3'-fluoro-biphenyl-4'-yl]-benzene
Trifluoromethoxy-4-[-4-(hex-4-enyl)-3'-fluoro-biphenyl-4'-yl]-benzene
Trifluoromethoxy-4-[-4-(hex-5-enyl)-3'-fluoro-biphenyl-4'-yl]-benzene
Trifluoromethoxy-4-[-4-(2-methylpropyl)-3'-fluoro-biphenyl-4'-yl]-benzene

Example 12

Trifluoromethoxy-2-fluoro-4-[4-methyl-2'-fluoro-biphenyl-4'-yl]-benzene is obtained analogously to example 15 from 4-methyl-2'-fluoro-4'-bromobiphenyl and 1-bromo-3-fluoro-4-(trifluoromethoxy)-benzene.

The following compounds are prepared analogously:
Trifluoromethoxy-2-fluoro-4-[4-ethyl-2'-fluoro-biphenyl-4'-yl]-benzene
Trifluoromethoxy-2-fluoro-4-[4-propyl-2'-fluoro-biphenyl-4'-yl]-benzene
Trifluoromethoxy-2-fluoro-4-[4-butyl-2'-fluoro-biphenyl-4'-yl]-benzene
Trifluoromethoxy-2-fluoro-4-[4-pentyl-2'-fluoro-biphenyl-4'-yl]-benzene
Trifluoromethoxy-2-fluoro-4-[4-hexyl-2'-fluoro-biphenyl-4'-yl]-benzene
Trifluoromethoxy-2-fluoro-4-[4-heptyl-2'-fluoro-biphenyl-4'-yl]-benzene
Trifluoromethoxy-2-fluoro-4-[4-methoxy-2'-fluoro-biphenyl-4'-yl]-benzene
Trifluoromethoxy-2-fluoro-4-[4-ethoxy-2'-fluoro-biphenyl-4'-yl]-benzene
Trifluoromethoxy-2-fluoro-4-[4-propoxy-2'-fluoro-biphenyl-4'-yl]-benzene
Trifluoromethoxy-2-fluoro-4-[4-butoxy-2'-fluoro-biphenyl-4'-yl]-benzene
Trifluoromethoxy-2-fluoro-4-[4-pentoxy-2'-fluoro-biphenyl-4'-yl]-benzene

Trifluoromethoxy-2-fluoro-4-[4-heptoxy-2'-fluoro-biphenyl-4'-yl]-benzene

Example 13

Trifluoromethoxy-4-[2-fluoro-4-ethoxybiphenyl-4'-yl]-benzene is prepared analogously to example 15 from 2-fluoro-4-ethoxy-4'-bromobiphenyl and 1-bromo-4-(trifluoromethoxy)-benzene.

The following compounds are prepared analogously:
Trifluoromethoxy-4-[2-fluoro-4-methylbiphenyl-4'-yl]-benzene
Trifluoromethoxy-4-[2-fluoro-4-ethylbiphenyl-4'-yl]-benzene
Trifluoromethoxy-4-[2-fluoro-4-propylbiphenyl-4'-yl]-benzene
Trifluoromethoxy-4-[2-fluoro-4-butylbiphenyl-4'-yl]-benzene
Trifluoromethoxy-4-[2-fluoro-4-pentylbiphenyl-4'-yl]-benzene
Trifluoromethoxy-4-[2-fluoro-4-hexylbiphenyl-4'-yl]-benzene
Trifluoromethoxy-4-[2-fluoro-4-heptylbiphenyl-4'-yl]-benzene
Trifluoromethoxy-4-[2-fluoro-4-methoxybiphenyl-4'-yl]-benzene
Trifluoromethoxy-4-[2-fluoro-4-propoxybiphenyl-4'-yl]-benzene
Trifluoromethoxy-4-[2-fluoro-4-butoxybiphenyl-4'-yl]-benzene
Trifluoromethoxy-4-[2-fluoro-4-pentoxybiphenyl-4'-yl]-benzene
Trifluoromethoxy-4-[2-fluoro-4-hexoxybiphenyl-4'-yl]-benzene
Trifluoromethoxy-4-[2-fluoro-4-heptoxybiphenyl-4'-yl]-benzene
Trifluoromethoxy-4-[2-fluoro-4-octoxybiphenyl-4'-yl]-benzene

Example 14

4-(Trifluoromethoxy)-2'-fluoro-4'-propylbiphenyl is obtained analogously to example 15 from 4-propyl-2-fluoro-bromobenzene and 1-bromo-4-(trifluoromethoxy)-benzene.

The following compounds are prepared analogously:
4-(Trifluoromethoxy)-2'-fluoro-4-methylbiphenyl
4-(Trifluoromethoxy)-2'-fluoro-4-ethylbiphenyl
4-(Trifluoromethoxy)-2'-fluoro-4-butylbiphenyl
4-(Trifluoromethoxy)-2'-fluoro-4-pentylbiphenyl
4-(Trifluoromethoxy)-2'-fluoro-4-hexylbiphenyl
4-(Trifluoromethoxy)-2'-fluoro-4-heptylbiphenyl
4-(Trifluoromethoxy)-2'-fluoro-4-octylbiphenyl
4-(Trifluoromethoxy)-2'-fluoro-4-nonylbiphenyl
4-(Trifluoromethoxy)-2'-fluoro-4-decylbiphenyl
4-(Trifluoromethoxy)-2'-fluoro-4-methoxybiphenyl
4-(Trifluoromethoxy)-2'-fluoro-4-ethoxybiphenyl
4-(Trifluoromethoxy)-2'-fluoro-4-propoxybiphenyl
4-(Trifluoromethoxy)-2'-fluoro-4-butoxybiphenyl
4-(Trifluoromethoxy)-2'-fluoro-4-pentoxybiphenyl
4-(Trifluoromethoxy)-2'-fluoro-4-hexoxybiphenyl
4-(Trifluoromethoxy)-2'-fluoro-4-heptoxybiphenyl
4-(Trifluoromethoxy)-2'-fluoro-4-octoxybiphenyl
4-(Trifluoromethoxy)-2'-fluoro-4-nonoxybiphenyl
4-(Trifluoromethoxy)-2'-fluoro-4-decoxybiphenyl

Examples of compositions

Example A

A.1. A liquid crystalline medium is prepared consisting of:
80.8 %

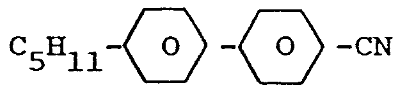

39.2 %

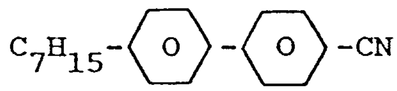

10 %

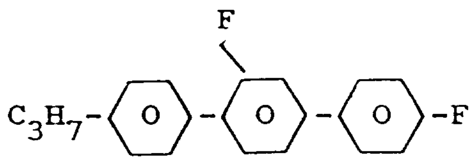

This mixture has a clearing point of N 42.4 - 42.5 °C I and a viscosity of 31.9 cSt at 20 °C.

A.2. A liquid crystalline medium is prepared consisting of compounds which are all known from the state of the art:

56.4 %

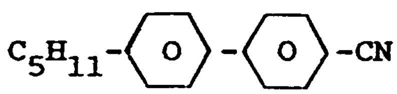

43.6 %

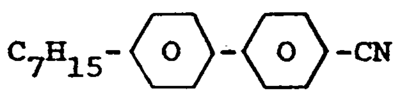

This mixture exhibits a clearing point of N 38 I and has a viscosity of 35 cSt at 20 °C.

A comparison of the mixtures of A.1. and A.2. clearly demonstrates the advantageous properties of the compounds according to formula I.

## Claims

1. Liquid crystalline medium being a mixture of at least two compounds, characterized in that at least one compound is a fluorinated oligophenyl according to formula I

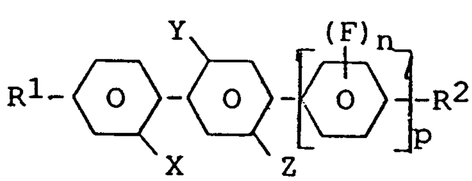

I

wherein
one of the groups $R^1$ and $R^2$ is F, $OCF_3$, $OCHF_2$ or NCS and the other group of the two groups $R^1$ and $R^2$ are independently from each other alkyl with 1 to 14 C atoms, wherein 1 or 2 $CH_2$ groups can be replaced by -O-, - O-CO-, -CO-O- and/or -CH=CH- with the proviso that two O atoms are not directly linked,
X, Y and Z each denote independently from each other H or F,
n is 0 or 1,
p is 0, 1 or 2 with the provisos that in case p =0, X is F and that in case p = 1 or 2, at least one of the groups X, Y and Z is F,

**2.** Fluorinated oligophenyls according to formula I

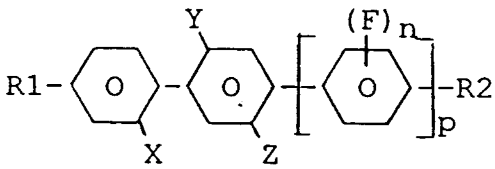

I

the meaning of X, Y, Z, n, p, $R^1$ and $R^2$ being indicated in claim 1.

**3.** Liquid crystal display device, characterized in that it contains a liquid crystalline medium according to claim 1.

**4.** Electrooptical display device, characterized in that it contains a liquid crystalline medium according to claim 1.

**Patentansprüche**

**1.** Flüssigkristalline Medien, bei denen es sich um ein Gemisch aus mindestens 2 Verbindungen handelt, dadurch gekennzeichnet, daß mindestens eine Verbindung ein fluoriertes Oligophenyl gemäß Formel I ist,

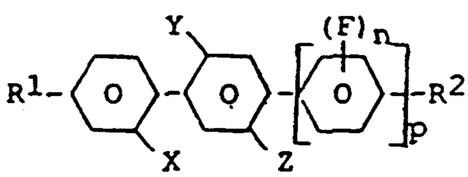

worin
eine der Gruppen $R^1$ und $R^2$ F, $OCF_3$, $OCHF_2$ oder NCS und die andere der beiden Gruppen $R^1$ und $R^2$ unabhängig von der ersten Gruppe Alkyl mit 1 bis 14 C-Atomen, worin 1 oder 2 $CH_2$-Gruppen durch -O-, -O-CO-, -CO-O- und/oder -CH=CH- ersetzt sein können, mit der Maßgabe, daß zwei O-Atome nicht direkt miteinander verknüpft sind,
X, Y und Z jeweils unabhängig voneinander H oder F, n 0 oder 1 und
p 0, 1 oder 2 bedeuten, mit den Maßgaben, daß bei p gleich 0 X F und daß bei p gleich 1 oder 2 mindestens eines aus der Gruppe X, Y und Z F bedeuten.

**2.** Fluorierte Oligophenyle gemäß Formel I

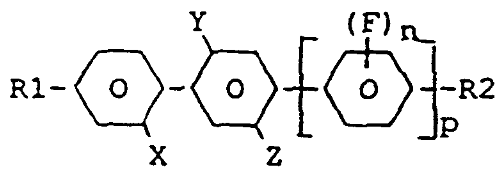

I

wobei die Bedeutung von X, Y, Z, n, p, $R^1$ und $R^2$ in Anspruch 1 angegeben ist.

**3.** Flüssigkristall-Anzeigevorrichtung, dadurch gekennzeichnet, daß sie ein flüssigkristallines Medium nach Anspruch 1 enthält.

**4.** Elektrooptische Anzeigevorrichtung, dadurch gekennzeichnet, daß sie ein flüssigkristallines Medium nach Anspruch 1 enthält.

**Revendications**

**1.** Milieu cristallin liquide qui est un mélange d'au moins deux composés, caractérisé en ce qu'au moins un composé est un oligophényle fluoré selon la formule I :

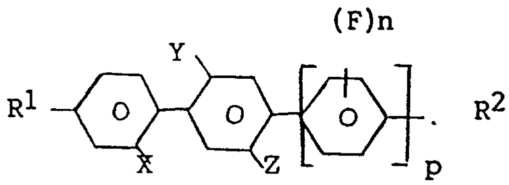

dans laquelle
une des radicaux $R^1$ et $R^2$ est F, $OCF_3$, $OCHF_2$ ou NCS et l'autre radical des deux radicaux $R^1$ et $R^2$ sont indépendamment l'un de l'autre un alkyle avec 1 à 14 atomes de carbone, dans lequel un ou deux groupes $CH_2$ peut être remplacé par -O-, -O-CO-, -CO-O- et /ou -CH=CH-, à la condition que deux atomes O ne soient pas directement liés, X, Y, et Z représentent indépendamment les uns des autres H ou F,
n est 0 ou 1,
p est 0, 1 ou 2, aux conditions que dans le cas où p = 0, X soit F et dans le cas où p = 1 ou 2, au moins l'un parmi X, Y et Z soit F.

**2.** Oligophényles fluorés selon la formule I

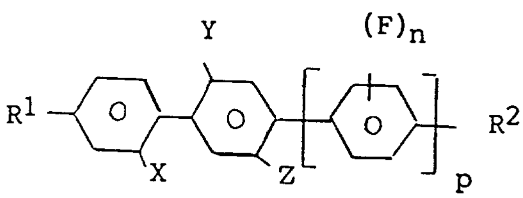

la signification de X, Y, Z, n, p, $R^1$ et $R^2$ étant indiquée dans la revendication 1.

**3.** Dispositif d'affichage à cristaux liquides, caractérisé en qu'il contient un milieu cristallin liquide selon la revendication 1.

**4.** Dispositif d'affichage électro-optique, caractérisé en qu'il contient un milieu cristallin liquide selon la revendication 1.